# EUROPÄISCHE PATENTSCHRIFT

(11) **EP 0 706 416 B1**
(45) Veröffentlichungstag und Bekanntmachung des Hinweises auf die Patenterteilung: **03.09.1997**
(21) Anmeldenummer: 94923688.9
(22) Anmeldetag: 27.06.1994
(51) Int. Cl.: B01D 3/34, C07C 29/80

(54) **VERFAHREN FÜR DIE DESTILLATIVE RÜCKGEWINNUNG VON GLYKOLEN AUS GEBRAUCHTEN GEFRIERSCHUTZMITTELN ODER KÜHLERSCHUTZMITTELN**
DISTILLATIVE METHOD OF RECOVERING GLYCOLS FROM USED ANTIFREEZE AGENTS
PROCEDE DE RECUPERATION DE GLYCOLS PAR DISTILLATION A PARTIR D'ANTIGELS OU DE LIQUIDES DE REFROIDISSEMENT USAGES

(30) Priorität: 01.07.1993 DE 4321846
(43) Veröffentlichungstag der Anmeldung: 17.04.1996
(73) Patentinhaber: BASF AKTIENGESELLSCHAFT, 67056 Ludwigshafen (DE)
(72) Erfinder: SCHNEIDER, Kurt, D-67098 Bad Dürkheim (DE)
(86) Internationale Anmeldenummer: EP9402084
(87) Internationale Veröffentlichungsnummer: WO9501213

(56) Entgegenhaltungen:
- Keine einschlägigen Dokumente gefunden

## Beschreibung

Die vorliegende Erfindung betrifft ein Verfahren für die destillative Rückgewinnung von Glykolen aus gebrauchten Gefrierschutzmitteln oder Kühlerschutzmitteln.

Gebrauchte Gefrierschutzmittel oder Kühlerschutzmittel aus Verbrennungsmotoren fallen jährlich in großen Mengen an. Da diese Flüssigkeiten erhebliche Mengen - bis zu 50 Vol.-% - an Glykolen, insbesondere an Ethylenglykol und Propylenglykol und darüber hinaus eine Reihe von Zusatzstoffen, wie Korrosionsinhibitoren oder Entschäumer (vgl.: Ullmanns Enzyklopädie der Technischen Chemie, 4. Auflage, Band 12, S. 208 bis 209, Verlag Chemie, Weinheim 1976) enthalten, können diese gebrauchten Gefrierschutzmittel oder Kühlerschutzmittel nicht einfach ins Abwasser gegeben werden, sondern müssen gesondert entsorgt oder aufgearbeitet werden. Zur Entsorgung der Gefrierschutzmittel oder Kühlerschutzmittel durch Verbrennung muß infolge ihres hohen Wassergehaltes eine erhebliche Energie aufgewendet werden, weshalb diese Art der Entsorgung kostspielig und unwirtschaftlich ist.

Aus diesem Grunde wurde eine Reihe von Verfahren zur destillativen Wiedergewinnung von Glykolen aus gebrauchten Gefrierschutzmitteln oder Kühlerschutzmitteln ausgearbeitet, beispielsweise die Verfahren gemäß DE-A 40 30 331 oder US-A 4 080 247. Bei der destillativen Aufarbeitung gebrauchter Gefrierschutzmittel oder Kühlerschutzmittel tritt das Problem auf, daß durch die thermische Zersetzung stickstoffhaltiger Zusatzstoffe während der Destillation erhebliche Mengen gasförmiger Stickoxide NOₓ freigesetzt werden, die aus ökologischen Gründen nicht an die Luft abgegeben werden können. Aus diesem Grunde mußten bislang die so freigesetzten Stickoxide, beispielsweise mittels Wasserringpumpen, abgesaugt und das derart belastete Abwasser nach Neutralisation in der Kläranlage gereinigt werden. Infolge des kostenträchtigen, erheblichen technischen Aufwandes, der zur Entfernung der freigesetzten Stickoxide nach diesem Verfahren betrieben werden muß, ist die Rückgewinnung von Glykolen aus gebrauchten Gefrierschutzmitteln oder Kühlerschutzmitteln bislang unwirtschaftlich geblieben.

Der vorliegenden Erfindung lag deshalb die Aufgabe zugrunde, ein wirtschaftliches Verfahren für die destillative Rückgewinnung von Glykolen aus gebrauchten Gefrierschutzmitteln oder Kühlerschutzmitteln zu finden, das nicht die vorgenannten Nachteile hat.

Nach langem Bemühen und nach Prüfung einer Vielzahl chemischer Substanzen wurde schließlich ein

Verfahren für die destillative Rückgewinnung von Glykolen aus gebrauchten Gefrierschutzmitteln oder Kühlerschutzmitteln gefunden, das dadurch gekennzeichnet ist, daß man das gebrauchte Gefrierschutzmittel oder Kühlerschutzmittel, die Stickstoffhaltige Zusatzstoffe enthalten, vor und/oder während dessen destillativer Aufarbeitung mit einer wirksamen Menge Amidosulfonsäure behandelt.

Es wurde gefunden, daß durch die erfindungsgemäße Behandlung der Gefrierschutzmittel oder Kühlerschutzmittel mit Amidosulfonsäure der Formel I

H₂NSO₃H I

die Bildung von Stickoxiden bei der destillativen Aufarbeitung von Gefrierschutzmitteln oder Kühlerschutzmitteln vollständig unterbunden werden kann.

Zur Behandlung der gebrauchten Gefrierschutzmittel oder Kühlerschutzmittel mit Amidosulfonsäure wird das gebrauchte Schutzmittel im allgemeinen bei Temperaturen von 0 bis 150°C, vorzugsweise 20 bis 100°C und besonders bevorzugt bei 50 bis 80°C mit im allgemeinen 1 bis 20, vorzugsweise mit 5 bis 15 und besonders bevorzugt mit 5 bis 10 g Amidosulfonsäure pro Liter gebrauchtem Schutzmittel behandelt. Die Behandlung des gebrauchten Schutzmittels kann sowohl in einer der Destillation vorausgehenden, gesonderten Behandlungsstufe als auch während der Destillation erfolgen. Im letzteren Fall wird die Amidosulfonsäure zum zu destillierenden Gefrierschutzmittel oder Kühlerschutzmittel gegeben und dieses destilliert. Vorteilhaft wird die optimal zuzusetzende Menge für einzelne Chargen gebrauchten Gefrier- oder Kühlerschutzmittels in einem einfachen Vorversuch ermittelt.

Vorteilhaft wird die erfindungsgemäße Vorbehandlung der gebrauchten Gefrier- oder Kühlerschutzmittel bei niedrigen pH-Werten des Schutzmittels durchgeführt, im allgemeinen bei pH-Werten von 1 bis 6, vorzugsweise bei pH-Werten von 2 bis 5,5 und besonders bevorzugt bei pH-Werten von 3 bis 4. Der pH-Wert des gebrauchten Gefrier- oder Kühlerschutzmittels kann beispielsweise durch die Zugabe einer entsprechenden Menge an Amidosulfonsäure eingestellt werden, vorteilhaft säuert man das gebrauchte Schutzmittel mit einer nichtflüchtigen, nichtoxidierenden Mineralsäure, z.B. mit Phosphorsäure oder Schwefelsäure, bis zum gewünschten pH-Wert an und setzt anschließend die zur Verhinderung der Stickoxidbildung erforderliche Menge an Amidosulfonsäure dem gebrauchten Gefrier- oder Kühlerschutzmittel zu.

Die Dauer dieser Vorbehandlung ist von der gewählten Behandlungstemperatur abhängig. Bei niedrigen Temperaturen ist im allgemeinen eine längere Behandlungsdauer erforderlich als bei erhöhter Temperatur.

Die Vorbehandlung erfordert keinerlei besondere Apparate. Sie kann z.B. in vorteilhaft mit Rührwerken ausgestatteten Lagertanks, offenen Rührkesseln oder Rohrreaktoren durchgeführt werden.

Die destillative Rückgewinnung der Glykole aus den so vorbehandelten gebrauchten Gefrierschutzmitteln kann nach an sich gängigen Destillationsverfahren des Standes der Technik ausgeführt werden, beispielsweise nach den Verfahren von Ullmanns Enzyklopädie der Technischen Chemie, 4. Auflage, Band 8, S. 201 bis 202, Verlag Chemie, Weinheim 1974; DE-A 40 30 331, US-A 4 080 247 oder DE-A 23 64 151.

Das erfindungsgemäße Verfahren kann zur Rückgewinnung von Glykolen, insbesondere Ethylenglykol und Propylenglykol, aus praktisch allen handelsüblichen Gefrier- oder Kühlerschutzmitteln eingesetzt werden.

Die für das erfindungsgemäße Verfahren benötigte Amidosulfonsäure ist beispielsweise durch die Umsetzung von Harnstoff mit rauchender Schwefelsäure nach dem in Brauer, Handbuch der Präparativen Anorganischen Chemie, S. 455 bis 456, Enke, Stuttgart 1960 beschriebenen Verfahren erhältlich.

### Beispiel

2000 g eines gebrauchten, Ethylenglykol enthaltenden Kühlerschutzmittels mit einem Wassergehalt von ca. 60 Gew.-% wurden mit 16 g 85 %iger Phosphorsäure und 5 g Amidosulfonsäure bei 20°C ca. 5 bis 6 Stunden gerührt.
Anschließend wurde bei einem Druck von 200 bis 300 mbar das enthaltene Wasser möglichst vollständig abdestilliert.
Die so konzentrierte Ethylenglykollösung wurde dann bei einem Druck von 1 bis 10 mbar fraktioniert destilliert, wobei 590 g Ethylenglykol zurückgewonnen wurden.
Bei der Destillation entstanden keine Stickoxide.

## Patentansprüche

1. Verfahren für die destillative Rückgewinnung von Glykolen aus gebrauchten Gefrierschutzmitteln oder Kühlerschutzmitteln, dadurch gekennzeichnet, daß man das gebrauchte Gefrierschutzmittel oder Kühlschutzmittel, die Stickstoffhaltige Zusatzstoffe enthalten, vor und/oder während dessen destillativer Aufarbeitung mit einer wirksamen Menge Amidosulfonsäure behandelt.

2. Verfahren nach Anspruch 1, dadurch gekennzeichnet, daß man die Behandlung des gebrauchten Gefrierschutzmittels oder Kühlerschutzmittels mit Amidosulfonsäure bei einem pH-Wert von 1 bis 6 durchführt.

## Claims

1. A method for discovering glycols from used antifreeze compositions by distillation, wherein the used antifreeze composition comprising nitrogen containing additives is treated before and/or during its distillation with an effective amount of sulfamic acid.

2. A method as claimed in claim 1, wherein the treatment of the used antifreeze composition with sulfamic acid is carried out at pH 1-6.

## Revendications

1. Procédé de récupération par distillation de glycols à partir d'antigels ou de réfrigérants usagés, caractérisé en ce que l'on traite l'antigel ou le réfrigérant usagé, qui contiennent des additifs azotés, avec une quantité efficace d'acide amidosulfonique avant et/ou pendant leur régénération par distillation.

2. Procédé selon la revendication 1, caractérisé en ce que l'on mène le traitement de l'antigel ou du réfrigérant usagé avec de l'acide amidosulfonique à un pH de 1-6.
